# EUROPEAN PATENT APPLICATION

(11) **EP 3 789 046 A1**
(43) Date of publication of application: **10.03.2021**
(21) Application number: 19195765.3
(22) Date of filing: 05.09.2019
(51) Int. Cl.: A61L 15/46, A01N 55/02, A61K 6/69, A61K 8/19, A61L 12/08, A61L 26/00, A61L 27/54, A61L 29/16, A61L 31/16

(54) **BIOCOMPATIBLE NANOPARTICLES WITH VARIOUS SURFACE FUNCTIONALIZATION FOR ANTIMICROBIAL APPLICATIONS**

(71) Applicant: Nanordica Medical Oü, 13628 Tallinn (EE)
(72) Inventor: KUBO, Anna-Liisa, 13628 Tallinn (EE); VASILIEV, Grigory, 13628 Tallinn (EE); BONDARENKO, Olesja, 13628 Tallinn (EE)
(74) Representative: Sarap, Margus

(57) **Abstract**

Present invention is related to the functionalization of antimicrobial metal-based nanoparticles with the negatively charged ligands (such as carboxyl or polyethylene glycol) that increase their biocompatibility. An antibacterial metal-based nanoparticles (such as CuO and Ag) with certain surface functionalization (such as COOH and polyethylene glycol) are used in antibacterial products to decrease nanoprticle cytotoxicity to human cells and, thus, to increase the biocompatibility. Nanoparticles according to invention are safer to the human cells and to the environment compared to unfunctionalized CuO nanoparticles and especially Ag nanoparticles, since they are less toxic to human cells and do not cause inflammation in the immune cells and have good antibacterial properties.

## Description

### Technical Field

Present invention is related to the functionalization of antimicrobial metal-based nanoparticles with the negatively charged ligands (such as carboxyl or polyethylene glycol) that increase their biocompatibility.

### Background Art

The number of bacteria resistant to antibiotics is increasing continuously. Alternatives to the usage of classical antibiotics are antibacterial metal ions, metal oxides and nanoparticles of thereof. The most widely used antibacterial metal is currently silver (or nanoparticles of thereof) that is used in patches, plasters, surgical dressings, surfaces, sprays, catheters, ointments, drops etc. However, recent scientific publications showed that silver and especially silver-based nanoparticles can be harmful to the environment and to the human cells, and some silver-containing products were banned from the market.

Copper is a versatile alternative to silver. It has excellent antibacterial properties and is less harmful to the environment and to the human cells compared to silver. However, similarly to Ag nanoparticles, also CuO nanoparticles can induce some adverse effects in human cells.

Present invention is related to the functionalization of antimicrobial metal-based nanoparticles (such as Ag and CuO nanoparticles) with negatively charged ligands (such as carboxyl and polyethylene glycol) to decrease nanoparticle cytotoxicity to human cells. As the proof of concept, we demonstrated that CuO functionalized with carboxyl groups (CuO-COOH) or polyethylene glycol (CuO-PEG) were significantly less toxic to human cells *in vitro* than traditional unfunctionalized CuO nanoparticles and nanoparticles functionalized with the positively charged amino groups (CuO-NH₂).

Specifically, we demonstrated that unfunctionalized CuO nanoparticles as well CuO-NH₂ nanoparticles were toxic for skin cell model HACAT (24-h EC₅₀=21.7 mg/1) and caused inflammation in immune cell model (differentiated THP-1), whereas CuO-COOH and CuO-PEG did not. Furthermore, CuO-COOH and CuO-PEG nanoparticles (NPs) were significantly more toxic to bacteria *Escherichia coli* than to human cells *in vitro,* providing better therapeutic window than unfunctionalized CuO nanoparticles or CuO-NH₂ nanoparticles. CuO-COOH nanoparticles were also effective in killing bacteria, when incorporated into plaster. In addition, CuO-COOH nanoparticles enhanced the wound healing as demonstrated with fibroblast migration assay. Thus, unfuctionalized CuO nanoparticles are less suitable for use in the medical products that interact closely with the human skin cells or immune cells compared to CuO-COOH and CuO-PEG nanoparticles that can be advised as antibacterials, since they were significantly more toxic to bacteria *Escherichia coli* than to human cells *in vitro,* did not induce inflammation and enhanced wound healing.

Increasing resistance of bacteria to conventional antibiotics necessitates the development of new antimicrobials, such as silver and copper-based products, including in nano-formulations. Silver is historically the most used antimicrobial metal, and colloidal silver nanoparticles were registered as a biocidal material in the US since 1954¹. Currently, silver nanoparticles (NPs) are the most commercialized NPs being in over 350 consumer products² but recent research data showed that silver nsnopsrticles can have (eco)toxic effects, and certain silver nanoparticles-containing products were banned from the market by the United States Environmental Protection Agency (US EPA)³. Thus, more safe alternatives to silver nanoparticles are urgently needed.

Copper is another metal with antibacterial effect that is used to inhibit bacterial spreads by employing Cu or CuO nanoparticles (NPs) on surfaces⁴, in suspension⁵ and in textiles⁶. Previous studies have shown that CuO NPs supported the wound healing⁷ and bone regeneration⁸. Given the mentioned properties, CuO NPs are ideal candidates for the use in medicines as wound dressings, antibacterial disinfectants, antimicrobial plasters or internal implants by combining two functions: antimicrobial activity and increased wound healing or osteogenesis. However, in case of biomedical use as antibacterials, CuO NPs may induce cytotoxicity, since they will contact closely with the human cells such as skin keratinocytes (in case of wound dressings or suspensions) and immune cells (in case of implants) residing in the blood and tissues. Warningly, the antibacterial concentrations of CuO NPs were demonstrated in the range of 20 - 280 mg/l, implying that the therapeutic value of the existing (mostly unfunctionalized) CuO NPs as antibacterials is rather limited, since the CuO NPs effective in killing bacteria are, similarly to silver NPs, also toxic to the human cells *in vitro*^{9,10}.

Functionalization of synthetic nanoparticles with various surface groups enables to modulate the interaction of nanoparticles with cells, and thus, change the safety profiles of nanoparticle. The introduction of functional groups enables to modify the surface charge of nanoparticles that changes the uptake of nanoparticles by cells and the toxicity of nanoparticles.

This is the first report on the antimicrobial toxicity *vs* safety towards human cells of CuO NPs with different surface functionalizations: unfunctionalized CuO, CuO-NH₂, CuO-COOH, CuO-PEG NPs and CuSO₄ as an ionic control.

### Summary of invention

Our invention is the usage of antibacterial metal-based NPs (such as CuO and Ag) with certain surface functionalization (such as COOH and polyethylene glycol) in antibacterial products to decrease NP cytotoxicity to human cells and, thus, to increase the biocompatibility. Our nanoparticles are safer to the human cells and to the environment compared to unfunctionalized CuO NPs and especially Ag NPs, since they are less toxic to human cells (Figure 1-2), do not cause inflammation in the immune cells (Figure 3) and have good antibacterial properties (Figures 4-6).

### Brief description of the drawings

The present invention will be described in detail in the following description with references to the accompanied drawings.

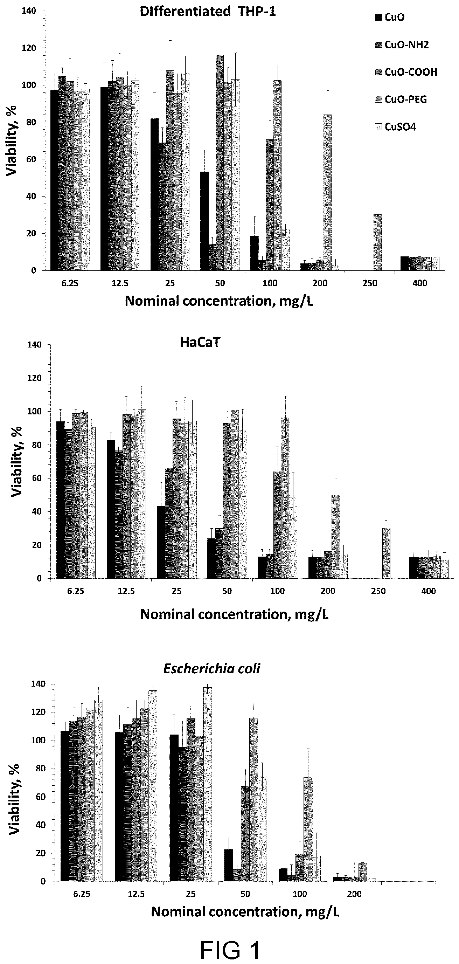

Fig 1 illustrates viability (Alamar Blue assay) of differentiated THP-1 (A), HACAT keratinocytes (B) and *Escherichia coli* (C) after 24-h incubation with CuO nanoparticles and CuSO₄ in RPMI medium supplemented with 10% fetal bovine serum;

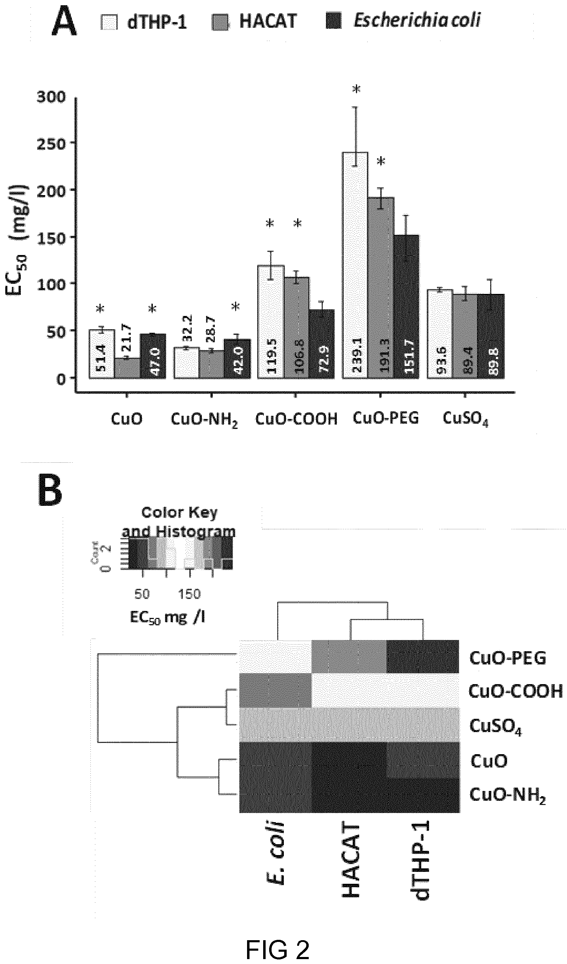

Fig 2 illustrates the average nominal 24-h EC₅₀ values (mg/1) with 95% confidence intervals (A) and dendrogram showing the clustering of average nominal 24-h EC₅₀ values of Cu compounds to bacteria *Escherichia coli,* HACAT keratinocytes and differentiated THP-1 cells (dTHP-1) (B). CuO-COOH and CuO-PEG are less toxic to bacteria than to human cells *in vitro;*

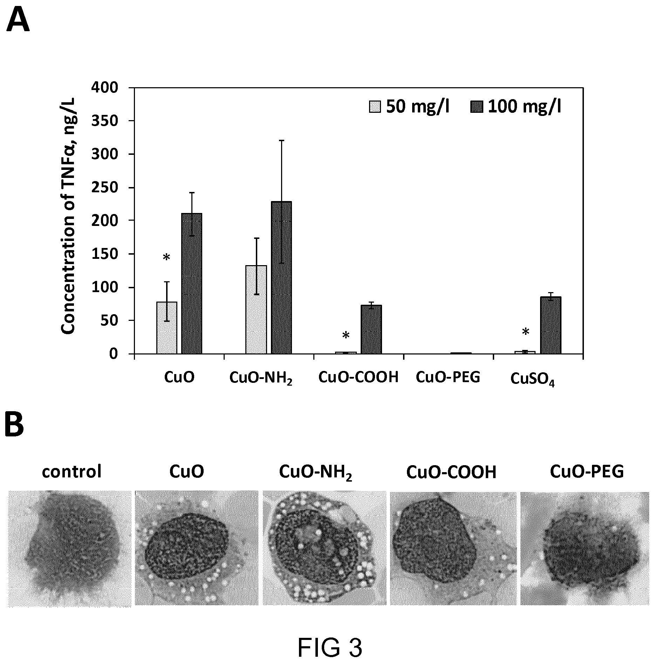

Fig 3 illustrates a concentration of TNF-α in the supernatants of differentiated THP-1 cells exposed to Cu compounds for 24 h. CuO-COOH NPs induce low inflammation and CuO-PEG no inflammation at tested concentrations (A). Vascularization of the THP-1 cells after exposure to Cu compounds (B).

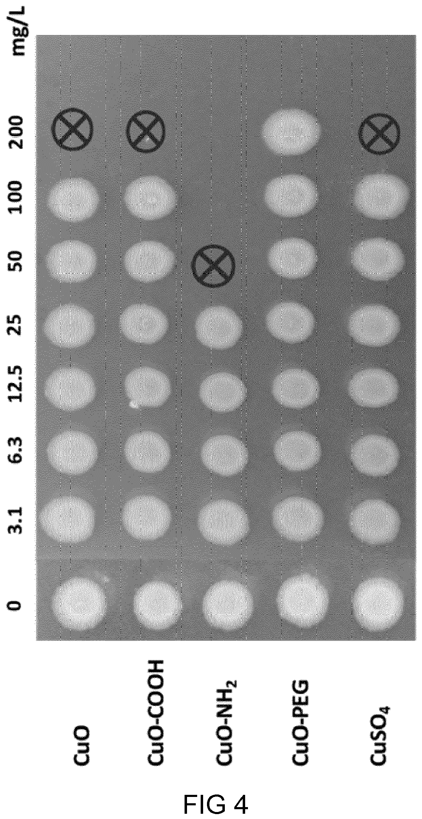

Fig 4 illustrates the antibacterial assay showing the minimal bactericidal concentration (MBC, designated as the ring with a cross) of CuO nanoparticles and CuSO₄.

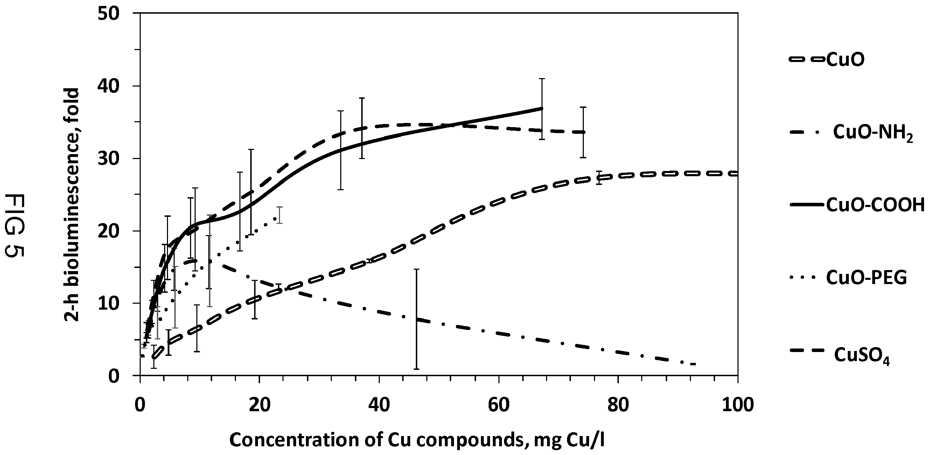

Fig 5 illustrates an induction of bioluminescence in *Escherichia coli* biosensor in response to Cu compounds (bioavailability of Cu from Cu compounds to bacteria).

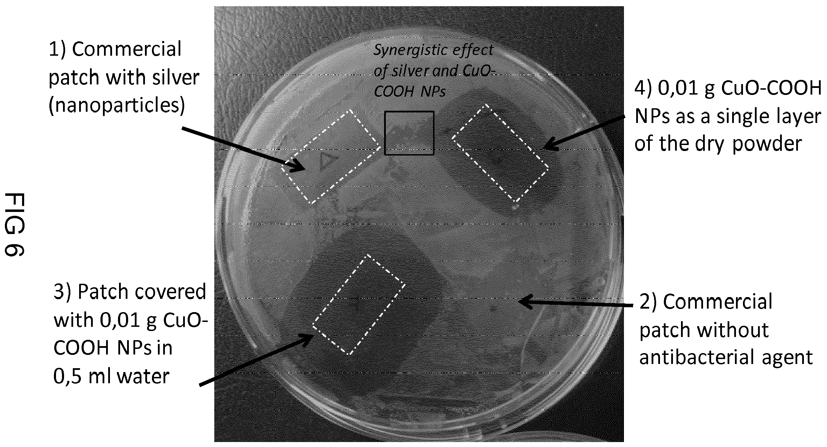

Fig 6 illustrates an agar-diffusion assay showing antibacterial properties of CuO-COOH nanoparticles against *Escherichia coli* on prototype plaster (patch area 2.5 cm²) impregnated with 0.5 ml 20 mg/ml nanoparticles' solution (X), covered with 20 mg dry nanoparticles powder (□) as compared to the commercially available Hansaplast silver plaster (Δ) and the commercial plaster without nanoparticles (*). Patch area is specified in white and was comparable in case of all prototype, showing that the prototype with CuO-COOH nanoparticles had the largest zone of bacterial inhibition and thus, better antibacterial properties compared to commercial silver-based patch.

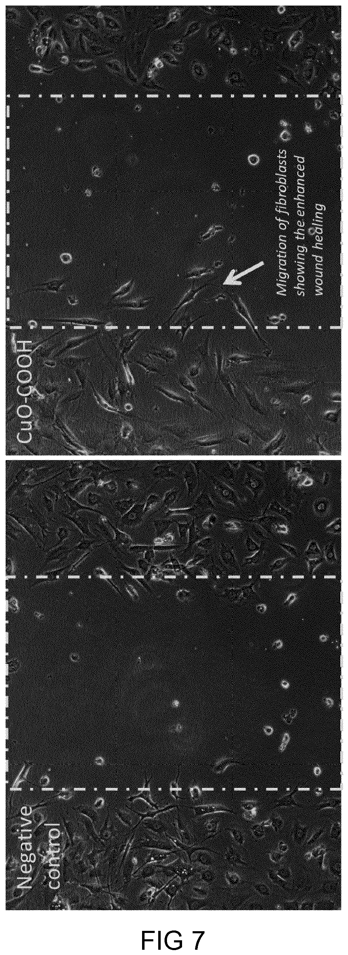

Fig 7 illustrates migration of Balb/c T3T fibroblasts (a model for connective tissues cells) exposed to the 25 mg/l CuO-COOH nanoparticles and demonstrates the enhanced wound healing induced by CuO-COOH CuO-COOH.

### Description of embodiments

Detailed description is divided into 3 parts: a) description of the physico-chemical properties of nanoparticles; b) comparison of the effects of nanoparticles on human and bacterial cells; c) efficiency (antibacterial effect) of nanoparticles and their use in prototype products (exemplified by prototype plaster).

### a) Nanoparticles and their physico-chemical properties

Four types of differently functionalized CuO NPs are employed in this patent description. The invention relates to the biocompatible properties of CuO-COOH and CuO-PEG nanoparticles. The other Cu compounds (CuO, CuO-NH₂ and CuSO₄) are described for comparison. The CuO NPs were synthetized by decomposition of Cu₂CO₃(OH)₂, followed by the introduction of the surface groups *via* treatment with mercaptopropionic acid (described previously in patent US2011/0252580A1) resulting in CuO-S-CH₂-CH₂-NH₂ (further referred as CuO-NH₂), CuO-S-CH₂-CH₂-COOH (CuO-COOH) and CuO-S-CH₂-CH₂-COO-PEG (Mw of PEG=750 g/mol)-OCH₃ (CuO-PEG).

Hydrodynamic size (Dh), polydispersity index (pdi) and zeta potential (Z-potential) were measured in 100 mg/l suspensions in DI water or cell culture medium using Malvern zetasizer (Zetasizer Nano-ZS, Malvern Instruments, UK).

Endotoxin content in CuO dispersions was assessed using the chromogenic limulus amoebocyte lysate (LAL) assay (Charles River Endosafe, Charleston, SC) according to the manufacturer's instructions and was found to be below the detection limit.

Cu content was determined using total reflection X-ray fluorescence (TXRF, Picofox S2, Bruker Corporation) from 100 mg/l suspensions.

The primary size of CuO NPs was below 1000 nm, and the special surface area was greater than 60 m²/cm³. Hydrodynamic size of NPs was in the range of 204 nm (CuO NPs) to 1268 nm (CuO-PEG). The Z-potential reflecting the particle charge was positive for CuO and CuO-NH₂ and negative for CuO-COOH and CuO-PEG in MQ water. Measured total Cu content was the highest for CuO (76.8%), followed by CuO-NH₂ (46.2%), CuO-COOH (33.6%) and CuO-PEG (11.7%). The measured total Cu content in CuSO₄ was 37.1± 4.5 % being in agreement with the calculated amount of Cu in CuSO₄ (39.8%) and Cu content in CuO was 76.8 ± 5.7 % being in agreement with the calculated amount of Cu in CuO (79.9%).

Present invention comprises antibacterial metal-based nanoparticles (in our case copper but can be also other metals with antibacterial properties and similar metal chemistry such as silver, zinc, iron, cobalt, titanium or their oxides or their combinations) functionalized with ligand, wherein the ligand has a formula of **X-R-Y,** whereas **X** is attached to the nanoparticle (in our case -SH (thiol group) but can be also phosphonate, sulfonate, carboxylate, dithiocarboxylate, sulfonic acid, phosphonic acid, carboxylic acid, dithiocarboxylic acid, phosphonate, sulfonate, carboxylate, dithiocarboxylate or amine), **R** is connecting X and Y (in our case R is -CH₂-CH₃, (alkyl group) but can be also aryl, citrate, carbohydrate, amine, vinyl, oligomer, polymer or protein) and **Y** is providing a biological function as for example reducing the adverse biological effect of nanoparticle to human cells or/and increasing the antibacterial effect, whereas in our case Y is carboxyl but can be also alcohol, amine, thiol, azide, quarternary amine, vinyl sulfone, sulfonic acid, phosphonic acid, dithiocarboxylic acid, alkyl, aryl, vinyl, citrate, carbohydrate, oligomer, carboxylic acid, polymer, peptide, protein, phage, antibody or polyoxometalate, SH, OH, NH₂, or CO₂H. The new biological biological functions of such functionalized nanoparticle construct are described in details in the sections below.

**Table 1. Physico-chemical characteristics of Cu compounds.**

| Cu compounds | Hydrodynamic diameter (Dh) in DI water¹ nm (pdi²) | Dh in test medium¹, nm (pdi) | Z-potential in DI water¹, nm | Cu content³, % |
|---|---|---|---|---|
| CuO NPs | 237 ± 31 (0.25) | 204 ± 13 (0.45) | 27.5 ± 1.8 | 76.8 ± 5.7 |
| CuO-NH₂ NPs | 733 ± 252 (0.24) | 936 ± 229 (0.67) | 25.8 ± 1.3 | 46.2 ± 4.0 |
| CuO-COOH NPs | 1124 ± 128 (0.35) | 303 ± 84 (0.70) | -12.0 ± 22 | 33.6 ± 3.2 |
| CuO-PEG NPs | 1244 ± 254 (0.35) | 1268 ± 315 (0.88) | -21.9 ± 3.3 | 11.7 ± 1.0 |
| CuSO₄ | NA | NA | NA | 37.1 ± 4.5 |

| | | | | |
|---|---|---|---|---|
| ¹Measured by Malvern Zetasizer from 100 mg/l suspensions; ²Polydispersity index ^{:3}Analyzed by TXRF from 100 mg/l suspensions. | | | | |

### b) Toxicity testing of the Cu compounds

All the human cell lines were obtained from American Type Culture Collection (ATCC) and cultured according to ATCC guidance. The human monocytic leukemia cell line THP-1 (ATCC TIB-202) was grown in Roswell Park Memorial Institute medium with L-glutamine (RPMI-1640, Corning) supplemented with 10% fetal bovine serum (FBS, Corning), 100 mM sodium pyruvate solution (Na-Pyr, Gibco) and 1% solution containing 10000 U/ml Penicillin and 10000 µg/ml Streptomycin (PEST, Gibco) that is further referred as the complete cell culture medium (CCM). Before the assays, THP-1 cells were differentiated into macrophage like cells by culturing them with 100 ng/ml phorbol myristate acetate (PMA, Invivogen) in CCM for 3 days. The human HACAT cell line, immortalized keratinocytes were obtained from ATCC PCS-200-011) and cultured according to ATCC guidance.

*Escherichia coli* MG1655 (obtained from the *E. coli* genetic stock centre, Yale University) and recombinant bioluminescent *E. coli* MC1061 (pSLcueR/pDNPcopAlux) were stored on agarized Luria-Bertani medium (LB, 1% tryptone, 0.5% yeast extract, 0.5% NaCl, 1.5% agar) and before the toxicity tests cultivated in 3 ml of LB medium at 30°C with shaking at 200 rpm overnight.

For the toxicity assay with bacteria, *E. coli* cells were grown in LB medium overnight followed by removal of LB by centrifugation, resuspension of bacterial cells in CCM without PEST to ∼5x10⁵ colony forming units (CFU/ml), mixing 1:1 with CuO suspensions/CuSO₄/CCM in 96-well plates and incubation for 24 h at 37°C. Bacterial viability was estimated using Alamar Blue assay (Applichem, final concentration of 150 µg/mL) by measuring the excitation at 530 nm and emission at 590 nm. The metabolic activity (viability) of exposed cells was expressed in % by comparing their fluorescence with that of the untreated cells. The EC₅₀ values were calculated using MS Excel macro Regtox. To assess possible interference of NPs, NPs were incubated abiotically with the Alamar Blue and showed no unspecific reactions.

For the toxicity assay with human cells, cell culture medium was removed, cells were washed with phosphate-buffered saline (PBS) and handled as described above for bacterial cells.

Viability of cells measured by Alamar Blue assay after 24-h exposure to CuO NPs and CuSO₄ is shown in Figure 1. Clear dose-response to all compounds in all cell types was observed. At nominal concentrations, unfunctionalized CuO and especially CuO-NH₂ NPs were the most toxic to all cell types having significant effect on cellular viability already at concentrations of 25-50 mg/l.

Figure 2A depicts the average nominal 24-h EC₅₀ values calculated on the basis of the dose-response curves from Figure 1. Figure 2B is a dendrogram showing the clustering of these EC₅₀ values. The EC₅₀ values were calculated using MS Excel macro Regtox and the results were presented with 95% confidence intervals. The heatmap and dendrogram were done with R Language and Environment for Statistical Computing.

When the 24-h EC₅₀ values of CuSO₄ were very similar for all cell types, the toxicity of NPs to different cells varied significantly. Namely, unfunctionalized CuO and CuO-NH₂ were more toxic to human cells *in vitro* than to bacteria, whereas negatively charged NPs - CuO-COOH and CuO-PEG - were significantly more toxic to bacteria compared to human cells (Figure 2A). Dendrogram analysis pointed out several clusters: the most toxic NPs - uncoated CuO and CuO-NH₂ - clustered together, whereas CuO and CuSO₄ formed another cluster and the least toxic CuO-PEG NPs a separate cluster (Figure 2B). Surprisingly, the most common unfunctionalized CuO NPs were almost as toxic as CuO-NH₂ NPs to human cells *in vitro* (Figure 2A), especially for skin cells HACAT (24-h EC₅₀=21.7 mg/l, the lowest toxicity value obtained in this study). It was previously demonstrated that 24-h EC₅₀ of unfunctionalized CuO NPs to HACAT was around 30 mg/l (MTT reduction assay), and that CuO induced ROS, oxidative stress, DNA damage and apoptosis in HACAT cells¹². Thus, unfuctionalized CuO NPs seem to be not suitable for use in the medical products that interact closely with the human skin cells or immune cells. Instead CuO-COOH and CuO-PEG NPs can be advised as antibacterials, since they were significantly more toxic to bacteria than to human cells *in vitro.*

### Ability of Cu compounds to induce inflammation in THP-1 cells

Differentiated THP-1 cells were exposed to CuO NPs and CuSO₄. After 24-h exposure supernatants were collected, centrifuged for 10 min at 10 000 x g. TNF-α was measured on 96-well plates using ELISA kit (Invitrogen 88-7346) according to manufacturer's instructions.

For the automatic photographing, THP-1 cells were differentiated in 24-well plates, exposed to NPs (24-h EC₂₀ concentrations), washed, stained with Giemsa Stain (Sigma-Aldrich) according to manufacturer's instructions and visualized using Automated Digital Morphology System CellaVision®.

ELISA test revealed that CuO-NH₂ and also CuO NPs were the most potent inducers of TNF-α (an inflammation marker) in differentiated THP-1 cells inducing TNF-α already at 50 mg/l, whereas CuO-COOH and CuSO₄ induced slight TNF-α production starting from 100 mg/l and CuO-PEG did not induce TNF-α at tested concentrations (Figure 3A).

Using the light microscopy we observed extensive vacuolization in the cells exposed to CuO and especially CuO-NH₂ NPs (Figure 3B) that was previously suggested to be a sign of inflammation and cell death. This vascularization was not seen in case of CuO-COOH and CuO-PEG, confirming the biocompatibility of these nanoparticles.

### Minimal bactericidal concentration (MBC) of the Cu compounds to bacteria

*Escherichia coli* were exposed to the test compounds in 96-well microtiter plates in LB medium at 37°C for 24 hours in the dark with minor shaking after every 15 min. After incubation, 3 µl of the sample was pipetted onto the LB agar plates and incubated at 30°C for 24 hours. The concentration of the chemical that yielded no visible growth/colonies of exposed bacteria on the agar plates was defined as MBC. Three independent experiments were performed.

Figure 4 illustrates the MBC of the Cu compounds, demonstrating that CuO-COOH NPs are as effective antibacterial as CuO.

### Determination of bioavailable fraction of Cu

Quantification of intracellular Cu ions was performed using recombinant biosensor bacteria *E. coli* MC1061 (pSLcueR/pDNPcopAlux) in which Cu ion-inducible promoter *copA* is genetically coupled to the bioluminescence-encoding genes *luxCDABE.* Thus, bioluminescence of this recombinant *E. coli* increases in response to sub-toxic concentrations of intracellular Cu ions in a dose-dependent manner. In the toxic concentration range, the bioluminescence of bacteria gradually decreases.

Figure 5 illustrates that CuO-COOH NPs were as effective as CuSO₄ in induction of biosensor, showing that the Cu from these NPs is highly bioavailable to bacteria and is effective in killing bacteria.

### Testing of prototype plaster

The agar-diffusion assay with prototype plaster was done according to ISO 20645¹¹ using *Escherichia coli* K12 (ATCC10798). *E. coli* bacteria was plated on the Petri dish with the nutrient agar. Then, 3 plasters (patches) and the powder of CuO-COOH NPs were applied on the Petri dish as follows: 1) commercial silver plaster; 2) commercial plaster without antibacterial agent, and 3) plaster impregnated CuO-COOH NPs (the cloth area of the prototype plaster was impregnated with CuO-COOH nanoparticles soaking with nanosuspensions for 1 min and drying). As a positive control, the dry powder of CuO-COOH NPs was also applied directly (4). The absence of bacterial growth (inhibition zones) after incubation with 3 patches and CuO-COOH NP as powder was estimated by visual inspection.

Figure 6 shows the antibacterial potential of CuO-COOH incorporated in prototype plasters against *E. coli.* The nanoparticles were delivered to the cloth area of the plasters in suspensions or as dry powders. CuO-COOH nanoparticles had the largest zone of bacterial inhibition and thus, overperformed the commercial silver-based plaster (Figure 6). This can be a result of solubility (Table 1) and higher bioavailability of CuO-COOH nanoparticles (Figure 5) to bacteria. Interestingly, we observed an additional unexpected bacteria-free area (marked with black square on Figure 6) between the commercial silver patch and dry CuO-COOH NP as powder, demonstrating a synergistic effect between Ag and CuO-COOH NPs and suggesting that their combinations could be used as even more efficient antibacterial agent. Also, these NPs could be used in combinations with the other antimicrobial agents such as quaternary ammoniums, surfactants, ethanol, chloroxine, antibiotics etc.

### Migration of fibroblasts Balb/c T3T in vitro (wound healing assay)

Balb/c T3T fibroblasts fibroblasts (a model for connective tissues cells crucial in the wound healing process) were let to form a confluent monolayer on 24-well plate. After that, the Newborn calf serum (NBCS) was withdrawn from the medium for 24 h. After 24-h, the cell monolayer was scratched in a straight-line using a 200 µl pipette tip to mimic an incisional wound. Cells were then washed with PBS to remove debris and treated with CuO-COOH and incubated at 37 °C with the medium containing 1% NBCS. Plates with cells were photographed after 24 h.

Figure 7 illustrates the migration of Balb/c T3T exposed to the CuO-COOH nanoparticles but not in the control. This demonstrates that CuO-COOH can enhance the migration of fibroblast and thus, facilitate the wound healing.

In addition, our preliminary data showed that CuO-COOH nanoparticles are taken up by mammalian cells (HACAT and differentiated macrophages) at orders of magnitude higher degree compared to unfunctionalized CuO nanoparticles and co-localize in the lysosomes/phagosomes with bacteria. Thus, these nanoparticles can be used for efficient drug delivery into the cells and for the killing of internal bacteria and other pathogens, e.g. fungi and parasites.

Thus, our nanoparticles are efficiently killing bacteria, in the test tubes/plates (Figures 1-2 and 4) and also as a part of plaster prototype (Figure 6), do not cause inflammation in the immune cell model (Figure 3), are less toxic than the traditionally used silver and unfunctionalized CuO nanoparticles (Figures 1-2) and facilitate the wound healing (Figure 7). Thus, these NPs and the NPs with the similar composition and biological function can be successfully used in different antimicrobial application and products as exemplified below.

### Application areas and the use of the nanoparticles and wound care products

1) Antimicrobial function. The nanoparticles are effective against bacteria, fungi, protozoa, viruses, prions and pathological proteins (α-synuclein, amyloid). These nanoparticles kill pathogens, thus they can be used to reduce the source of infection on the skin and mucous membranes, in fresh and chronic wounds, as well as in the places of introduction of medical devices, in the places of operations and in the places of implants.
2) Regeneration function. These nanoparticles help connective tissue cells (fibroblasts, keratinocytes etc) grow, accelerate wound re-epithelization, help revitalize damaged tissue, reduce oxidative stress, restore normal cell structure after damage, reduce the effects of aging due to human nutrition with copper as an essential trace element, increasing tissue concentration of vascular endothelial growth factor (VEGF), platelet-derived growth factor (PDGF), nerve growth factors (NGFs), angiogenin, matrix metalloproteinases (MMP-1, -2, and -9 etc), copper peptide GHK, fibrinogen which are necessary to restore the tissues.
3) Anti-inflammatory function. Due to the p. 1 and 2, the nanoparticles reduce inflammation in the area of damage and reduce the cascade of inflammatory reactions, and thereby reduce further destruction of the tissues of the organism. These NPs reduce the level of inflammatory factors (such as Tumor Necrosis Factor-α, inflammatory interleukines, cytokines and cyclooxygenases).
4) Anesthetic function. Due to the above properties, these nanoparticles reduce pain from the wound site.

### Usage of the nanoparticles

1. The nanoparticles can be used:
   A) as an antimicrobial component or coating in medical devices like stents, catheters, abdominal plugs, adhesive films, contact lenses, lens cases, fibrous wound dressings, cotton gauzes, bandages, wound healing products, injectors of drugs and other devices that pass through the skin or contact with the skin, as well as prostheses, spokes, screws and other implants introduced into the body;
   B) they can also be an antimicrobial component of the material printed on 3D printers quaternary (e.g., comprising alginate and chitosan polymers and 3D structures of them, gauzes, films or foams) or produced by other technologies and afterwards implanted into the body; part of the nanorobots and electronic devices injected into blood, organs or used on the body surface;
   C) as an antimicrobial component in creams, ointments, lotion, sprays, liquids, lipsticks and other cosmetics
   D) as an antibacterial component for liquids, gels, soaps, shampoos, and so on for washing and disinfecting hands, wounds, surfaces and items
   E) as a component in textiles to reduce the number of microorganisms and, thus, to reduce the unpleasant smell
   F) as an antimicrobial component used in dentistry for disinfection on antimicrobial component for dental materials.

In addition the nanoparticles according to present invention can be used as a regenerating component of moisturizers, in creams from wrinkles, in creams before tanning and after tanning, in means for improving scars, in hair dye and tattoos, in antioxidant products for skin, in scrubs; as an anti-inflammatory component in medicals for the treatment of local and systemic autoimmune and allergic diseases, including psoriasis, allergic and non-allergic dermatitis, acne, alginate pemphigus and pemphigoid and in medical preparations for local anesthesia, analgesia, termanesthesia.

### Comparison with existing alternatives

Because of higher safety to human cells and the environment and cheapness of the raw material (copper), described copper nanoparticles are better alternative compared to most widely used silver nanoparticles. Low toxicity to human cells allows to increase the concentration of copper nanoparticles several times compared to silver nanoparticles, which leads to greater antibacterial activity. Above-described surface functionalization with the negatively charged ligands (such as COOH and PEG described above) significantly increase the compatibility of these nanoparticles. In addition, we demonstrated that CuO-COOH has additional beneficial effect and facilitates the wound healing.

The second existing alternative to our invention is the application of classic antibiotics to the surface of the medical devices but this is not optimal. The resistance of microorganisms to antibiotics gives rise to concerns about the ineffectiveness of antibiotics in the future. Furthermore, solubility of antibiotics in the water environment results in rapid irradiation of the antibiotics from the surface of the medical device and a decrease the antibacterial effectiveness of the coating. Thus, traditional antibiotics cannot be employed in some devices, on the surfaces and in wound-care products.

Previous patents have described the usage of various copper compounds for antibacterial and wound healing applications. Most of the described compounds, however, are salts and not nanoparticles (WO2001028339A2, US2016220728A1, US10016525B2). The linker system for synthesizing CuO-COOH nanoparticles was described previously (US20110252580A1) with proposing the possible wound care applications. However, this patent focuses on the synthesis of the nanoparticles and do not elaborate on their biological effects and do not claim the use of these particles in antimicrobial wound care products and medical devices. The WO2016170152A1 patent describes the copper oxo-hydroxide nanoparticles and the possibility of using them for wound care purposes but the chemical composition of copper oxo-hydroxide is different from our metal (oxide)-based nanoparticles.

In addition, this patent describes the nanoparticles solely as the delivery system for Cu ions and do not elaborate on their biocompatibility aspects. In our invention, the ligand has a crucial biological role and function - reducing the toxicity of nanoparticles to human cells.

### Citation list:

1. Nowack, B., Krug, H. F. & Height, M. 120 years of nanosilver history: Implications for policy makers. Environ. Sci. Technol. (2011). doi:10.1021/es103316q
2. https://www.nanotechproject.org/.
3. Carbone, M., Donia, D. T., Sabbatella, G. & Antiochia, R. Silver nanoparticles in polymeric matrices for fresh food packaging. J. King Saud Univ. - Sci. 28, 273-279 (2016).
4. Rosenberg, M., Vija, H., Kahru, A., Keevil, C. & Ivask, A. Rapid in situ assessment of Cu-ion mediated effects and antibacterial efficacy of copper surfaces. Sci. Rep. (2018).
5. Bastos, C. A. P. et al. Ligand-Doped Copper Oxo-hydroxide Nanoparticles are Effective Antimicrobials. Nanoscale Res. Lett. (2018). doi:10.1186/s 11671-018-2520-7
6. Teli, M. D. & Sheikh, J. Modified bamboo rayon-copper nanoparticle composites as antibacterial textiles. Int. J. Biol. Macromol. 61, 302-307 (2013).
7. Borkow, G. et al. Molecular mechanisms of enhanced wound healing by copper oxide-impregnated dressings. Wound Repair Regen. 18, 266-275 (2010).
8. Shi, M. et al. Copper-doped mesoporous silica nanospheres, a promising immunomodulatory agent for inducing osteogenesis. Acta Biomater. 30, 334-344 (2016).
9. Bondarenko, O. et al. Toxicity of Ag, CuO and ZnO nanoparticles to selected environmentally relevant test organisms and mammalian cells in vitro: A critical review. Arch. Toxicol. 87, 1181-1200 (2013).
10. Bondarenko, O. M. et al. Multilaboratory evaluation of 15 bioassays for (eco)toxicity screening and hazard ranking of engineered nanomaterials: FP7 project NANOVALID. Nanotoxicology 10, 1229-1242 (2016).
11. Standardization, E. C. for. ISO 20645:2004 Textile fabrics -- Determination of antibacterial activity -- Agar diffusion plate test. (2005).
12. Alarifi, S., Ali, D., Verma, A., Alakhtani, S. & Ali, B. A. Cytotoxicity and genotoxicity of copper oxide nanoparticles in human skin keratinocytes cells. Int. J. Toxicol. (2013).

## Claims

1. A nanoparticle construct for use as an antibacterial medical construct, whereas said construct comprising of
- an antibacterial metal-based nanoparticle functionalized with the ligand, wherein the ligand has a formula of **X-R-Y,** whereas **X** is attached to the nanoparticle and is a phosphonate, sulfonate, carboxylate, dithiocarboxylate, sulfonic acid, phosphonic acid, carboxylic acid, dithiocarboxylic acid, phosphonate, sulfonate, thiol, carboxylate, dithiocarboxylate or amine and **R** consitsts of alkyl, aryl, citrate, carbohydrate, amine, vinyl, oligomer, polymer or protein and **Y** is providing a biological function as for example reducing the adverse biological effect of nanoparticle to human cells or/and increasing the antibacterial effect, whereas Y is an alcohol, carboxylic acid, amine, thiol, azide, quarternary amine, vinyl sulfone, sulfonic acid, phosphonic acid, dithiocarboxylic acid, alkyl, aryl, vinyl, citrate, carbohydrate, oligomer, H, OH, NH₂, or CO₂H, polymer, peptide, protein, phage, antibody or polyoxometalate.

2. The nanoparticle construct according to claim 1, wherein the primary average size of the an antibacterial metal-based nanoparticles is below 1000 nm or the special surface area is greater than 60 m²/cm³.

3. The nanoparticle construct according to claim 2, whereas an antibacterial metal-based nanoparticles consist of silver, copper, zinc, iron, cobalt, titanium or their oxides or their combinations.

4. The nanoparticle construct according to claims 1-3, whereas an antibacterial metal-based nanoparticle nanoparticles are used in combination with traditional antibacterial agents such as silver or silver nanoparticles, ethanol, chloroxine, antibiotics to enhance their antibacterial effect through the synergy.

5. The nanoparticle construct according claims 1-3 for use as an antimicrobial component or coating in medical devices like stents, catheters, abdominal plugs, adhesive films, contact lenses, lens cases, fibrous wound dressings, cotton gauzes, bandages, wound healing products, injectors of drugs and other devices that pass through the skin or contact with the skin, as well as prostheses, spokes, screws and other implants introduced into the body.

6. The nanoparticle construct according to claims 1-3 for use as an antimicrobial component of the material printed on 3D printers or produced by other technologies and afterwards implanted into the body; part of the nanorobots and electronic devices injected into blood, organs or used on the body surface

7. The nanoparticle construct according to claims 1-3 for use as a product for washing and disinfecting hands, wounds, surfaces and items.

8. The nanoparticle construct according to claims 1-3 for use as a component in textiles to reduce the number of microorganisms and reduce the unpleasant smell from the fabric.

9. The nanoparticle construct according to claims 1-3 for use as a component in dentistry for disinfection on antimicrobial component for dental materials.

10. The nanoparticle construct according to claims 1-3 for use as delivery of drugs into cells such as macrophages, skin cells and other mammalian cells and acting itself as the drug killing the intracellular pathogens such as bacteria, fungi.

11. The nanoparticle construct according to claims 1-3 for use as an anti-inflammatory component in medicals for the treatment of local and systemic autoimmune and allergic diseases, including psoriasis, allergic and non-allergic dermatitis, acne, pemphigus and pemphigoid.

12. The nanoparticle construct according to claims 1-3 for use as an antibacterial wound dressing, spray, cream, plaster, patch, ointments, lotion, liquid, lipsticks and other cosmetics, gel, soap, shampoo, moisturizer, in cream from wrinkles, in creams before tanning and after tanning, for improving scars, in hair dye and tattoos, in antioxidant products for the skins.

13. The nanoparticle construct according to claims 1-3 for use as the antimicrobial wound care product comprising of a functionalized nanoparticle construct deployed in wound dressings comprising alginate and chitosan polymers and 3D structures of them, gauzes, films or foams.

14. The nanoparticle construct according to claims 1-3 for use as a nanoparticles suspension comprising further at least one additional antimicrobial agent, quaternary ammoniums, surfactants, etc.

15. The nanoparticle construct according to claims 1-3 for use as the medical preparations for anesthesia, analgesia, termanesthesia.
